# EUROPÄISCHE PATENTSCHRIFT

(11) **EP 0 339 443 B1**
(45) Veröffentlichungstag und Bekanntmachung des Hinweises auf die Patenterteilung: **21.12.1994**
(21) Anmeldenummer: 89106970.0
(22) Anmeldetag: 19.04.1989
(51) Int. Cl.: C07K 15/00, C12N 5/00, C12N 15/00, C12P 21/00, G01N 33/60, G01N 33/577

(54) **Monoklonaler Antikörper zur selektiven immunologischen Bestimmung von intaktem Prokollagen Peptid (Typ III) und Prokollagen (Typ III) in Körperflüssigkeiten**
Monoclonal antibody for the selective immune determination of intact procollagen peptide (type III) and procollagen (type III) in body fluids
Anticorps monoclonal pour la détermination immunologique sélective du peptide (type III) intact de procollagène et de procollagène (type III) dans des sécrétions

(30) Priorität: 27.04.1988 DE 3814216
(43) Veröffentlichungstag der Anmeldung: 02.11.1989
(73) Patentinhaber: HOECHST AKTIENGESELLSCHAFT, 65926 Frankfurt am Main (DE)
(72) Erfinder: Brocks, Dietrich, Dr., D-6200 Wiesbaden 42 (DE); Günzler-Pukall, Volkmar, Dr., D-3550 Marburg (DE); Hachmann, Henning, Dr., D-6000 Frakfurt am Main 71 (DE); Pünter, Jürgen, Dr., D-6238 Hofheim am Taunus (DE); Timpl, Rupert, Dr., D-8035 Gauting (DE)

(56) Entgegenhaltungen:
- EP-A- 0 089 008
- BIOLOGICAL ABSTRACTS, Band 83, Nr. 6, 1987, Seite AB-267, Zusammenfassung Nr.52561, Philadelphia, US; H. KONDO: "Establishment of mouse monoclonal antibodies against amino terminal peptide of type III procollagen and their use for a histochemical study in the human liver", & JPN J. GASTROENTEROL 83(10): 2174-2180, 1968
- CHEMICAL ABSTRACTS, Band 97, Nr. 1, 5. Juli 1982, Seite 444, ZusammenfassungNr. 4431s, Columbus, Ohio, US; N. SUNDARRAJ et al.: "Development andcharacterization of monoclonal antibodies to human type III procollagen",BIOCHEM. BIOPHYS. RES. COMMUN. 1982, 106(1), 48-57
- CLINICAL CHEMISTRY, Band 31, Nr. 8, 1985, Seiten 1301-1304; O. NIEMELÄ:"Radioimmunoassays for type III procollagen animo-terminal peptides in humans"

## Beschreibung

Prokollagen Peptid (Typ III) (P III P) ist das aminoterminale Propeptid des Kollagen (Typ III), das nach Sezernierung des Prokollagen (Typ III)-Moleküls extrazellulär abgespalten wird. Prokollagen-Peptid (Typ III) seinerseits läßt sich mit Kollagenase weiter zu Bruchstücken Col 1, Col 2 und Col 3 spalten, welche mit Hilfe von an sich bekannten proteinchemischen Methoden isoliert werden können (Nowack, H. et al., Eur. J. Biochem. 70, 205 - 216 (1976), Bruckner, P. et al., Eur. J. Biochem. 90, 595 - 603 (1978)).

Mit einer radioimmunologischen Bestimmungsmethode, wie sie in der Europäischen Patentschrift Nr. 4940 beschrieben ist, kann die Konzentration dieses Prokollagen Peptids in Körperflüssigkeiten bestimmt werden. Die Kenntnis der Serumkonzentration des Peptids erlaubt Aussagen über die Aktivität fibrotischer Erkrankungen, wie z.B. der Leber [Rohde, H. et al. Eur. J. Clin. Invest. 9, 451-459 (1979)].

Eine exakte, selektive Bestimmung von Prokollagen Peptid (Typ III) und Prokollagen (Typ III) in Serum und anderen Körperflüssigkeiten wurde in der Europäischen Patentanmeldung 289 930 vorgeschlagen. Dabei ist jedoch eine Reihe von Zentrifugationsschritten erforderlich, die den Einsatz dieses Tests im Routinelabor erschweren. Eine einfachere Handhabung erlaubt das Testverfahren des Immunoradiometric Assay (IRMA). Bei diesem Verfahren werden zwei Antikörper verwendet, von denen einer an einen festen Träger gekuppelt ist, wodurch ein Fällungsschritt und damit ein Zentrifugations- und einige Pipettierschritte entfallen.

Aus der Publikation von SundarRaj et al., 1982, Biochem. Biophys. Res. Commun, Vol. 106 (1), S 48-57, sind monoklonale Antikörper gegen menschliches Prokollagen Typ III bekannt. Diese reagieren jedoch - im Gegensatz zu den erfindungsgemäßen Antikörpern - mit Abbauprodukten des Prokollagens oder mit Kollagen Typ III (von Kollagen Typ III ist das aminoterminale Prokollagen Peptid Typ III abgespalten). Keiner der von SundarRaj beschriebenen monoklonalen Antikörper ist gegen ein Epitop des intakten, aminoterminalen Prokollagen Peptids Typ III gerichtet.

Der von Kondo et al. (Biolog. Abstracts, Band 83, Nr. 6, 1987, S. AB-267, Zusammenfassung Nr. 52561) beschriebene monoklonale Antikörper ist gegen das Peptid Col 2 gerichtet. Das aminoterminale Prokollagen Peptid Typ III enthält die Fragmente Col 1-3. Col 2 ist kleiner als Col 1. Der von Kondo et al. isolierte Antikörper reagiert nicht mit Col 1 oder einem Abbauprodukt von aminoterminalem Prokollagen Peptid Typ III, das größer als Col 1 ist.

Überraschenderweise wurde nun ein monoklonaler Antikörper gefunden, der mit einem Fragment des Prokollagen (Typ III) reagiert, das bisher in der Gelfiltrationschromatographie nicht als eigener Peak erkennbar war. In Kombination mit anderen monoklonalen oder polyklonalen Antikörpern mit Spezifität für Prokollagen Peptid (Typ III) und/oder Prokollagen (Typ III) erlaubt dieser Antikörper die Bestimmung dieser Antigene mittels der IRMA-Technik.

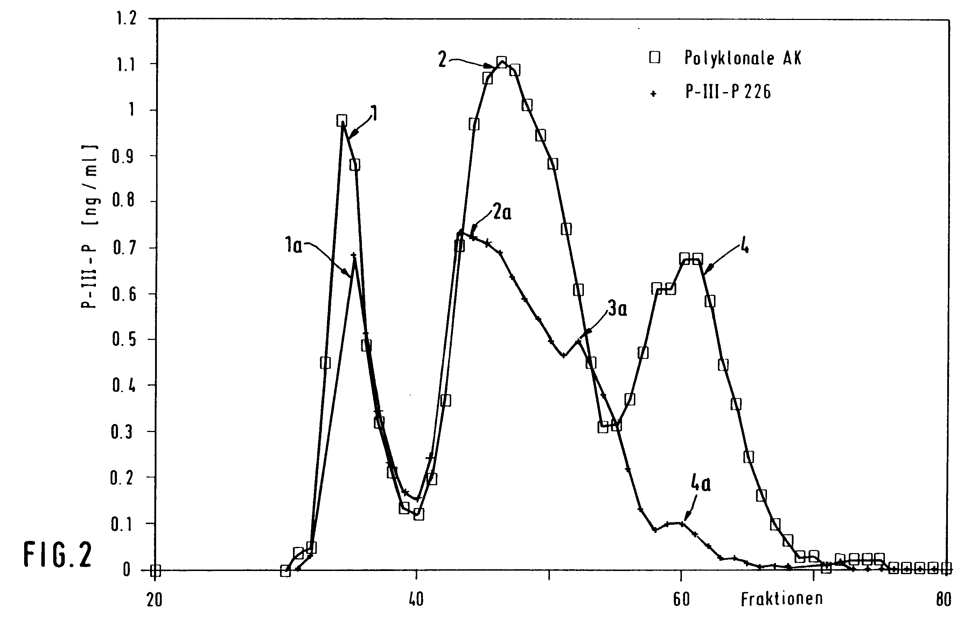

Die Erfindung betrifft somit:
1. Einen monoklonalen Antikörper mit dem in Fig. 2 dargestellten Reaktionsmuster gegenüber intaktem Prokollagen (Typ III) und pN-Kollagen (Prokollagen, dem das C-terminale Propeptid fehlt) (Peak 1a), intaktem aminoterminalen Prokollagen Peptid (Typ III) (Peak 2a), Abbauprodukten des aminoterminalen Prokollagen Peptids (Typ III), die mit ihrem Molekulargewicht zwischen dem des aminoterminalen Prokollagen Peptids (Typ III) und dem des Col 1 liegen (Peak 3a), sowie Col 1 und Abbauprodukten des aminoterminalen Prokollagen Peptids (Typ III) mit gleichem Molekulargewicht wie Col 1 (Peak 4a).
2. Eine Hybridoma Zellinie, die durch Fusion von Zellen aus einer Myelom Linie und Lymphozyten von einem zuvor mit Prokollagen Peptid (Typ III) immunisierten Tier gewonnen wird und die den unter 1. charakterisierten Antikörper produziert.
3. Ein Verfahren zur Herstellung des unter 1. charakterisierten Antikörpers.
4. Ein Verfahren zur quantitativen immunologischen Bestimmung von Prokollagen Peptid (Typ III) mit dem unter 1. charakterisierten Antikörper.
5. Eine diagnostische Zusammensetzung zur Feststellung der Prokollagen Peptid (Typ III)-Menge in Körperflüssigkeiten, die aus einer wirksamen Menge des unter 1. charakterisierten monoklonalen Antikörpers alleine oder in Kombination mit anderen Antikörpern, insbesondere mit einer wirksamen Menge des in der Europäischen Patentanmeldung 289 930 vorgeschlagenen monoklonalen Antikörpers, im Gemisch mit einem diagnostisch annehmbaren Träger besteht.

Im folgenden wird die Erfindung detailliert erläutert, insbesondere die bevorzugten Ausführungsformen. Ferner wird die Erfindung in den Patentansprüchen definiert.

Zur Herstellung der monoklonalen Antikörper können Tiere, bevorzugt Nagetiere, wie z.B. Mäuse, Ratten, Kaninchen, Meerschweinchen, mit Prokollagen Peptid (Typ III), das nach dem Verfahren des Europäischen Patents 4940 isoliert wurde, in Gegenwart von Adjuvans immunisiert werden. Besonders bevorzugt werden Mäuse eingesetzt, insbesondere solche vom SJL Stamm. Mit wiederholten Sekundärinjektionen, beispielsweise im Abstand von 4 bis 8 Wochen, wird die Immunantwort verstärkt. Der Erfolg der Immunisierung wird durch Bestimmung der Konzentration von Antikörpern im radiologischen Bindungstest [R. Timpl und L. Risteli, Immunochemistry of the extracellular matrix, H. Furthmayr Ed., Vol. 1, 199 (1982)] kontrolliert. Einige Tage vor der Fusion der Lymphozyten mit einer Myelom Zellinie werden die Tiere mit Prokollagen Peptid (Typ III) ohne Adjuvans behandelt. Lymphozyten der Tiere werden gewonnen und mit einer Myelom Zellinie fusioniert, die ebenfalls von einer der oben genannten Tierspezies stammen kann, vorzugsweise jedoch von der Maus, insbesondere mit der Zellinie P3X63AG8.653. Es werden vorteilhaft Lymphozyten mit Myelom Zellinien gleicher Spezies fusioniert. Die Fusion und die weitere Züchtung der Zellklone werden in einer dem Fachmann bekannten Weise durchgeführt, wobei im Überstand der Zellkulturen mittels immunologischer Bindungstests die Konzentration spezifischer Antikörper bestimmt wird. Aus den aus diesem Verfahren hervorgehenden Zellklonen wird ein Klon für die Verwendung im IRMA ausgesucht. Besonders bevorzugt wird mit einer Zellinie gearbeitet, die durch Fusion von Lymphozyten aus Prokollagen Peptid (Typ III)-immunisierten Mäusen des SJL-Stamms mit der Maus Myelom Zellinie P3X63AG8.653 hergestellt wird und einen monoklonalen Antikörper mit dem in Fig. 2 gezeigten Reaktionsmuster produziert. Diese Zellinie wurde am 2.3.1988 unter den Bedingungen des Budapester Vertrags bei European Collection of Animal Cell Cultures (ECACC), PHLS Centre for Applied Microbiology and Research, Porton Down, Salisbury, SP40JG, U.K. mit der Nummer 88030202 hinterlegt.

Die erfindungsgemäßen monoklonalen Antikörper gehören in die Klasse der IgG-, IgM- und IgA-Proteine. Antikörper der IgG Klasse, insbesondere der Subklasse IgG2b, sind besonders vorteilhaft einsetzbar. Verdeutlicht werden die Eigenschaften der monoklonalen Antikörper anhand des aus der Zellinie ECACC 88030202 gewonnenen monoklonalen Antikörpers PIIIP 226, wenn die im Serum vorhandenen Antigene über Gelfiltrationschromatographie nach ihrem Molekulargewicht aufgetrennt werden und die Fraktionen der Chromatographie im Radioimmunoassay eingesetzt werden. In Fig. 2 ist das Elutionsprofil der Gelfiltrationschromatographie, wie es der monoklonale Antikörper PIIIP 226 zeigt, im Vergleich zu dem Elutionsprofil, wie es polyklonale Antikörper aufweisen, gezeigt. Peak 1/1a entspricht intaktem Prokollagen (Typ III) bzw. pN-Kollagen (Typ III). Peak 2/2a entspricht intaktem aminoterminalen Prokollagen Peptid (Typ III) und Abbauprodukten hiervon mit ähnlicher Größe. Im Peak 3a werden Abbauprodukte des Prokollagen Peptids (Typ III) erfaßt, die im Chromatogramm einen kleineren Peak ergeben als Prokollagen Peptid (Typ III) jedoch einen deutlich größeren als Col 1 aufweisen. Das Molekulargewicht der Abbauprodukte liegt also jeweils zwischen ca. 45.000 (Prokollagen Peptid) und ca. 10.000 (Col 1). Der Peak 4/4a entspricht Col 1 sowie Abbauprodukten des aminoterminalen Prokollagen Peptids (Typ III) mit gleichem Molekulargewicht wie Col 1. Es zeigt sich, daß die Antigenfraktion, die bei der Analyse mit polyklonalen Antikörpern mit erfaßt wird und das Molekulargewicht des Abbauprodukts Col 1 hat bzw. Col 1 ist, von dem erfindungsgemäßen monoklonalen Antikörper deutlich schwächer erfaßt wird. Der erfindungsgemäße monoklonale Antikörper besitzt also eine spezifische Wirkung gegen ein Epitop des aminoterminalen Prokollagen Peptids (Typ III), das in Col 1 nicht vorhanden ist. Ferner wird ein Teil der Antigene, die von polyklonalen Antikörpern im Peak 2 erfaßt werden, vom Antikörper PIIIP 226 nicht erkannt, so daß an der Position des Peaks 2 mit dem Antikörper PIIIP 226 zwei Peaks (2a und 3a) erkennbar werden. Der Peak 3a wird mit dem in der Deutschen Patentanmeldung vorgeschlagenen Antikörper PIIIP 296 ebenfalls nicht gefunden.

Für die Herstellung der erfindungsgemäßen Antikörper ist es wichtig, daß eine geeignete Quelle zur Gewinnung des Antigens zur Verfügung steht. Wie schon erwähnt, wird humanes oder tierisches, hochgereinigtes Prokollagen Peptid (Typ III) vorteilhaft nach dem Verfahren des Europäischen Patents 4940 isoliert, indem Gewebe oder pathologische Körperflüssigkeiten mit Kollagenase abgebaut werden und das Prokollagen Peptid aus der Reaktionslösung abgetrennt und durch Kombination von chromatographischen Methoden und/oder Immunadsorption gereinigt wird.

Der erfindungsgemäße monoklonale Antikörper kann in verschiedenen immunologischen Verfahren, einschließlich aller Formen des Radioimmunoassay, z.B. sequentielle Sättigungsanalyse oder Gleichgewichtsanalyse sowie in anderen kompetitiven und nicht-kompetitiven Bindungsassays, wie Fluoreszenz-, Enzym-, Chemilumineszenz- oder anderen Immunoassays verwendet werden. Er ist vor allem zum Einsatz in Sandwichverfahren bei Immunosorbentassays geeignet. Der monoklonale Antikörper kann daher in immunologischen Verfahren zur Isolierung und Charakterisierung sowie zur quantitativen Bestimmung von Prokollagen Peptid (Typ III) in Geweben und Körperflüssigkeiten eingesetzt werden. Man verfährt nach dem Fachmann an sich bekannten Methoden, vorteilhaft indem eine flüssige Probe, die Prokollagen Peptid (Typ III) enthält, mit dem erfindungsgemäßen monoklonalen Antikörper, der an eine feste Matrix, die vorzugsweise aus Kunststoffmaterial besteht, insbesondere Kunststoffröhrchen, gekuppelt ist, zur Reaktion gebracht wird und die Menge des Prokollagen Peptids (Typ III) durch Bindung von polyklonalen oder eines zweiten monoklonalen Antikörpers, bevorzugt des in der Europäischen Patentanmeldung 289 930 vorgeschlagenen monoklonalen Antikörpers, der mit einer radioaktiven oder sonstigen Markierung versehen ist, bestimmt wird. Dieses Verfahren kann auch durchgeführt werden, indem polyklonale oder monoklonale Antikörper, insbesondere der in der Europäischen Patentanmeldung 289 930 vorgeschlagene monoklonale Antikörper, an eine feste Matrix gebunden und mit dem Antigen zur Reaktion gebracht werden, wobei dieses Antigen dann durch Bindung des markierten erfindungsgemäßen Antikörpers quantitativ bestimmt wird. Bei diesen Methoden spielt es keine Rolle, ob das Prokollagen Peptid (Typ III) noch mit dem Aminoterminus des Prokollagen (Typ III) verknüpft ist oder nicht. Die bislang bei der immunologischen Bestimmung mittels polyklonaler Antikörper störenden Degradationsprodukte des Prokollagen Peptid (Typ III), insbesondere das Col 1, werden von dem beschriebenen Testsystem nicht miterfaßt.

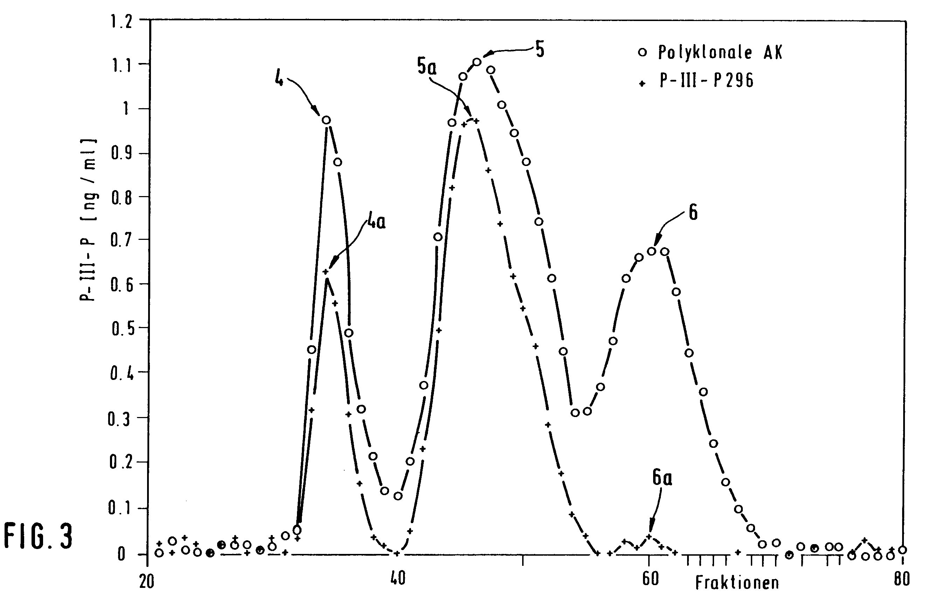

Der in der Europäischen Patentanmeldung 289 930 vorgeschlagene monoklonale Antikörper zeigt das in Figur 3 dargestellte Reaktionsmuster gegenüber intaktem Prokollagen (Typ III) und pN-Kollagen (Prokollagen, dem das C-terminale Propeptid fehlt, Peak 4a), intaktem aminoterminalen Prokollagen Peptid (Typ III) (Peak 5a) sowie Col 1 und Abbauprodukten des aminoterminalen Prokollagen Peptids (Typ III) mit gleichem Molekulargewicht wie Col 1 (Peak 6a).

Zur Herstellung dieses monoklonalen Antikörpers verfährt man im wesentlichen wie oben beschrieben. Besonders bevorzugt wird mit einer Zellinie gearbeitet, die durch Fusion von Lymphozyten aus Prokollagen Peptid (Typ III) immunisierten Mäusen des SJL-Stamms mit der Maus Myelom-Zellinie P3X63AG8.653 hergestellt wird. Diese Zellinie ist bei European Collection of Animal Cell Cultures (ECACC), PHLS Centre for Applied Microbiology and Research, Porton Down, Salisbury, SP40JG, U.K. unter der Nummer 87042308 hinterlegt.

In den folgenden Beispielen wird die Erfindung weitergehend erläutert. Prozentangaben beziehen sich auf das Gewicht, wenn nicht anders angegeben.

### Beispiel 1

### Herstellung von Prokollagen Peptid (Typ III)

Prokollagen-Peptid (Typ III) wird hergestellt durch Einwirkung von Kollagenase auf Prokollagen (Typ III) bei 37 °C. Hierbei wird das Peptid keinen Denaturierungsmitteln ausgesetzt. Zur Herstellung größerer Mengen des Peptids wird ein modifiziertes Verfahren angewendet. Alle Verfahrensschritte bis zur Einwirkung der Kollagenase werden im Kälteraum durchgeführt. Die verschiedenen NaCl-Lösungen, die zur Löslichmachung eingesetzt werden, enthalten 0,05 M Tris-HCl, pH 7,4, 0,01 M EDTA, Natriumazid (200 mg/ml) und die Protease-Inhibitoren Phenylmethylsulfonylfluorid (3 µg/ml) und p-Chlormercuribenzoat (3 µg/ml).

Foetale Kälberhaut (3 kg) wird in 10 l 1 M NaCl homogenisiert und zwei Tage extrahiert. Gelöstes Kollagen wird aus der Extraktionslösung gefällt durch Zugabe von festem NaCl bis zu einer Endkonzentration von 2,5 M. Nach Rühren über Nacht wird das Präzipitat durch Zentrifugation gesammelt (1800 x g, 20 Minuten), zweimal mit 2,5 M NaCl gewaschen und wieder gelöst, indem es über Nacht in 10 l 0,5 M NaCl gerührt wird. Kleine Mengen unlöslichen Materials werden durch Zentrifugation entfernt. Die so erhaltene Mischung von Kollagen (Typ III) und Prokollagen (Typ III) wird mit 1,6 M NaCl gefällt. Das Präzipitat wird dann in 2 l 0,05 M Tris-HCl (pH 8,0) suspendiert und nach Zusatz von 0,02 M CaCl₂ 20 Minuten auf 50 °C erhitzt und anschließend 3 Stunden bei 37 °C zusammen mit 1500 U bakterieller Kollagenase (CLSPA, Worthington, USA) pro Gramm feuchtes Präzipitat inkubiert. Nach Einwirkung der Kollagenase wird das gebildete Präzipitat durch Zentrifugation abgetrennt und die Lösung dialysiert gegen 0,005 M Tris-HCl, pH 8,0, 6,8 M Harnstoff und über eine DEAE-Cellulosesäule (5,0 x 30 cm) gegeben, die mit dem gleichen Puffer äquilibriert wurde.

Die auf der Säule gebundenen Proteine werden mit einer NaCl-Lösung ausgewaschen, deren Konzentration von 0 bis 0,3 M ansteigt. Die gesamte Elutionsmenge beträgt 2 l. Die aus der Säule ausfließende Lösung wird bezüglich der Absorption bei 236 nm und ihrer Antigenaktivität durch Verwendung von Antikörpern überprüft, die spezifisch für das aminoterminale Segment des Prokollagen (Typ III) sind. Normalerweise enthält der letzte Peak, der aus der Säule eluiert wird, das Prokollagen-Peptid (Typ III). Das Peptid wird durch Dialyse gegen destilliertes Wasser entsalzt und lyophilisiert. Die weitere Reinigung erfolgt auf einer Säule mit Agarose A 1,5 M (2 x 120 cm) (Fa. Biorad), die mit 1 M CaCl₂, 0,05 M Tris-HCl, pH 7,5 äquilibriert ist.

### Beispiel 2

### Hybridoma Herstellung

Mäuse vom SJL-Stamm werden mit 5 µg Prokollagen Peptid (Typ III), das nach Beispiel 1 erhalten wurde, in Gegenwart von komplettem Freund'schen Adjuvans intramuskulär immunisiert. Nach vier Wochen und nach drei Monaten wird die Immunreaktion durch eine weitere intramuskuläre Injektion von 5 µg Prokollagen Peptid (Typ III) in Gegenwart von inkomplettem Freund'schen Adjuvans verstärkt. Drei Tage vor der Fusion wird die Immunantwort durch Injektion von weiteren 50 µg Prokollagen Peptid (Typ III) verstärkt.

Zur Fusion werden die Tiere getötet und die Milzzellen isoliert. In Gegenwart von Polyethylenglykol werden die Milzzellen mit der Myeloma Zellinie P3X63AG8.653 fusioniert. (Die entstandene Zellinie ist bei der ECACC unter der Nummer 88030202 hinterlegt.) Durch Kultivierung der Fusionsmischung in Hypoxanthin-Aminopterin-Thymidin-Medium über einen Zeitraum von zwei Wochen wird auf Milzzell x P3X63AG8.653-Hybride selektioniert. Zur Erreichung einer monoklonalen Zellinie werden die erhaltenen Zellklone mehrfach subkloniert. Die Überstände der entstandenen Zellkolonien werden im radioimmunologischen Bindungstest auf Antikörperproduktion getestet. So erhält man den Bimonoklonalen Antikörper PIIIP 226.

### Beispiel 3

### Radioimmun-Bindungstest

300 µl Zellkulturüberstand oder eine andere Probe, wie z.B. Aszites nach Züchtung von Hybridom-Zellen in der Bauchhöhle von Mäusen, werden mit 100 µl einer ¹²⁵J-Prokollagen Peptid (Typ III) Lösung (1 ng Protein/100 µl, hergestellt wie in EP 4940, Beispiel 1, beschrieben) über Nacht inkubiert. Die gebildeten Antigen-Antikörper Komplexe werden durch Zugabe von Anti Maus IgG Serum vom Schaf oder einer anderen Spezies ausgefällt. Nach Zentrifugation und Dekantieren des Überstands wird die Menge der präzipitierten Radioaktivität im Gamma-Szintillationsspektrometer bestimmt.

### Beispiel 4

### Radioaktive Markierung der Antikörper

0,2 ml einer Lösung mit 0,2 mg des gemäß der Deutschen Patentanmeldung P 37 14 633.5, Beispiel 2, gewonnenen monoklonalen Antikörpers PIIIP 296 oder eines anderen Antikörpers in 0,05 M Phosphatpuffer, pH 7,4, werden in einem Polystyrol-Teströhrchen (12 x 55 mm) vorgelegt und mit 100 MBq Na¹²⁵J-Lösung, abgepuffert mit 10 µl 0,5 M Phosphatpuffer, pH 7,4, versetzt. Nach Zusatz von 50 µl einer wäßrigen Lösung von 20 µg Chloramin T wird 1 Minute gemischt. Anschließend wird die Jodierungsreaktion durch Zugabe von 50 µl einer wäßrigen Lösung von 20 µg Natriumdisulfit beendet. Das nicht umgesetzte Na¹²⁵J wird anschließend von dem ¹²⁵J-markierten Antikörper durch Chromatographie an einem Anionenaustauscher abgetrennt. Die chromatographischen Fraktionen, die den aufgereinigten ¹²⁵J-markierten Antikörper enthalten, werden mit einer Lösung aus 20 g Tween 20 und 14,6 g Na₂EDTA in einem Liter 0,05 M Tris-HCl, pH 8,0, verdünnt, so daß die Konzentration des markierten Antikörpers 200 µg/l beträgt.

### Beispiel 5

### Beschichtung von Teströhrchen mit den Antikörpern

Zur Fixierung von Antikörpern auf Polystyrol-Teströhrchen (12 x 75 mm) werden in jedes Röhrchen 300 µl einer Lösung von 4 mg/l Antikörper, z.B. PIIIP 226, in 0,01 M Natriumphosphatpuffer, pH 6,4, gegeben und über Nacht bei Raumtemperatur inkubiert. Anschließend wird die Antikörperlösung abgesaugt und in jedes Röhrchen 500 µl einer 1 %igen Lösung von Rinderserumalbumin in 0,05 M Tris-Citrat, pH 7,5, gegeben. Nach Inkubation über Nacht bei Raumtemperatur wird die Lösung abgesaugt. Die Antikörperbeschichteten Röhrchen werden über Silikagel getrocknet.

### Beispiel 6

### Immunoradiometrischer Test (Radioimmunometric Assay, IRMA)

0,1 ml der zu analysierenden Probe oder des Prokollagen Peptid (Typ III)-Standards werden in Polystyrol-Teströhrchen, die nach Beispiel 5 mit monoklonalem Antikörper PIIIP 226 beschichtet wurden, unter Zusatz von 0,1 ml Phosphat-gepufferter Saline (PBS) bei Raumtemperatur 2 Stunden inkubiert. Anschließend werden die Teströhrchen zweimal mit je 1 ml PBS gewaschen. Danach werden in die Röhrchen 200 µl ¹²⁵J-markierter Antikörper PIIIP 296 (= 40 ng Antikörper) oder eines anderen Antikörpers gegeben und 2 Stunden bei Raumtemperatur inkubiert. Die Radioaktivität der an die Röhrchenwand gebundenen Antikörper-Antigen-¹²⁵J-Antikörper-Komplexe wird nach zweimaligem Waschen mit je 1 ml PBS und anschließendem Dekantieren im Gamma-Szintillationsspektrometer bestimmt.

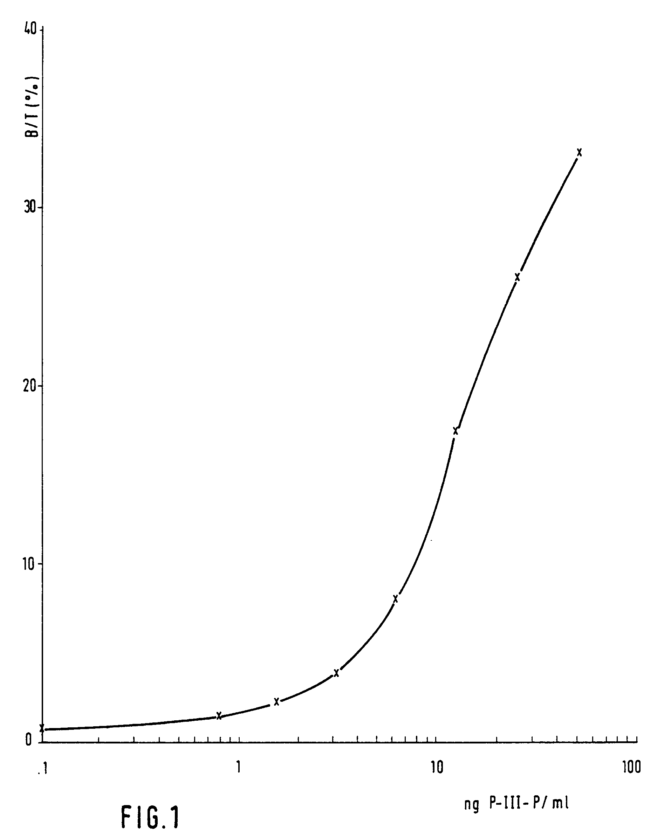

Durch Vergleich einer Eichkurve, zu deren Erstellung Standards mit unterschiedlichen Mengen Prokollagen Peptid (Typ III) eingesetzt werden, kann dann die Konzentration von Prokollagen Peptid (Typ III) in der unbekannten Probenlösung berechnet werden. Die Fig. 1 zeigt die Eichkurve des immunoradiometrischen Tests (B/T bedeutet Verhältnis von gebundener zu gesamt eingesetzter Radioaktivität).

### Beispiel 7

### Bestimmung der Molekulargewichtsverteilung der mit PIIIP 226 reagierenden Antigene in menschlichem Serum

1 ml Serum werden per Gelfiltrationschromatographie über ein Allyldextran, vernetzt mit N,N'-Methylenbisacrylamid [®Sephacryl S 300 Säule (1,6 x 130 cm)], äquilibriert in PBS mit 0,04 % eines nicht-ionischen Detergens, beispielsweise eines polyethoxylierten Sorbitan-Monolaurats (Tween 20), aufgetrennt. 0,2 ml jeder einzelnen Fraktion werden mit einer in Bezug auf die Menge des markierten Antigens limitierenden Menge des zu untersuchenden Antikörpers (in 0,1 ml Puffer) und 0,1 ml ¹²⁵J-markiertem Prokollagen Peptids (Typ III) (enthält 1 ng Protein) über Nacht bei 4 °C inkubiert. Anschließend wird mit einer vorher ausgetesteten Menge Anti-Maus IgG Serum vom Schaf in Gegenwart von 10 % Polyethylenglykol (PEG 6000) 1 Stunde inkubiert. Die gefällten Antigen-Antikörper-Komplexe werden abzentrifugiert (1500 x g) und nach Dekantieren wird im Gamma-Szintillationsspektrometer die Radioaktivität bestimmt. Durch Vergleich mit einer Eichkurve, zu deren Erstellung Standards mit unterschiedlichen Mengen Prokollagen Peptid (Typ III) eingesetzt werden, kann dann die Konzentration der mit dem eingesetzten Antikörper reagierenden Antigene in den Chromatographie-Fraktionen bestimmt werden. Fig. 2 zeigt das Elutionsprofil des mittels PIIIP 226 bestimmten Antigens im Vergleich zu dem Profil des mit Hilfe von polyklonalen Antikörpern bestimmten Antigens.

## Patentansprüche (Patentansprüche für folgende(n) Vertragsstaat(en): AT, BE, CH, DE, FR, GB, IT, LI, LU, NL, SE)

1. Monoklonaler Antikörper mit dem in Fig. 2 dargestellten Reaktionsmuster gegenüber intaktem Prokollagen (Typ III) und pN-Kollagen (Peak 1a), intaktem aminoterminalem Prokollagen Peptid (Typ III) (Peak 2a), Abbauprodukten des aminoterminalen Prokollagen Peptids (Typ III), die mit ihrem Molekulargewicht zwischen dem des aminoterminalen Prokollagen Peptids (Typ III) und dem des Col 1 liegen (Peak 3a), sowie Col 1 und Abbauprodukten des aminoterminalen Prokollagen Peptids Typ III mit gleichem Molekulargewicht wie Col 1 (Peak 4a).

2. Monoklonaler Antikörper nach Anspruch 1 mit spezifischer Wirkung gegen ein Epitop des aminoterminalen Prokollagen Peptids (Typ III), das in Col 1 nicht vorhanden ist.

3. Monoklonaler Antikörper nach Anspruch 1 oder 2, dadurch gekennzeichnet, daß er der Klasse IgG angehört.

4. Monoklonaler Antikörper nach einem oder mehreren der Ansprüche 1 bis 3, dadurch gekennzeichnet, daß er durch ein Hybridom gebildet wird, das durch Fusion von Zellen einer Myelom Linie mit Lymphozyten eines zuvor mit aminoterminalem Prokollagen Peptid (Typ III) immunisierten Tieres entstanden ist.

5. Monoklonaler Antikörper PIIIP 226 der Hybridoma Zellinie ECACC 88030202.

6. Hybridoma Zellinie, die einen Antikörper gemäß der Ansprüche 1 bis 5 produziert, gebildet durch Fusion von Zellen aus einer Myelom-Zellinie und Lymphozyten eines zuvor mit Prokollagen Peptid (Typ III) immunisierten Tieres.

7. Die Hybridoma Zellinie ECACC 88030202.

8. Ein Verfahren zur Herstellung eines monoklonalen Antikörpers nach einem oder mehreren der Ansprüche 1 bis 5, dadurch gekennzeichnet, daß
a) Tiere mit aminoterminalem Prokollagen Peptid (Typ III) immunisiert werden,
b) Lymphozyten gewonnen und mit Myelom-Zellen fusioniert werden,
c) die Hybride hinsichtlich des Vorliegens eines Antikörpers mit den in einem der Ansprüche 1 bis 3 angegebenen Eigenschaften ausgewählt und kloniert werden und
d) der Antikörper aus den genannten Klonen gewonnen wird.

9. Verfahren nach Anspruch 8, dadurch gekennzeichnet, daß zur Ausführung des Schritts d) die Hybridoma Zellinie ECACC 88030202 eingesetzt wird.

10. Verfahren zur quantitativen immunologischen Bestimmung von Prokollagen Peptid (Typ III) mit Antikörpern, dadurch gekennzeichnet, daß
a) eine flüssige Probe, die aminoterminales Prokollagen Peptid (Typ III) oder Prokollagen (Typ III) enthält, mit dem monoklonalen Antikörper nach einem oder mehreren der Ansprüche 1 bis 5 zur Reaktion gebracht wird und
b) die Menge des aminoterminalen Prokollagen Peptids (Typ III) bzw. des Prokollagens (Typ III) über den gebildeten Antigen-Antikörper-Komplex bestimmt wird.

11. Verfahren zur quantitativen immunologischen Bestimmung von Prokollagen Peptid (Typ III) mit Antikörpern, dadurch gekennzeichnet, daß
a) der monoklonale Antikörper nach einem oder mehreren der Ansprüche 1 bis 5 an eine feste Matrix gekuppelt wird,
b) eine flüssige Probe, die aminoterminales Prokollagen Peptid (Typ III) oder Prokollagen (Typ III) enthält, mit dem genannten Antikörper zur Reaktion gebracht wird und
c) das gebundene Antigen durch markierte monoklonale oder polyklonale Antikörper mit Spezifität für Prokollagen Peptid (Typ III) oder Prokollagen (Typ III) nachgewiesen wird.

12. Verfahren nach Anspruch 11, dadurch gekennzeichnet, daß in den Schritt a) polyklonale oder monoklonale Antikörper mit Spezifität für Prokollagen Peptid (Typ III) und/oder Prokollagen (Typ III) und in den Schritt c) der markierte monoklonale Antikörper nach einem oder mehreren der Ansprüche 1 bis 5 eingesetzt werden.

13. Verfahren nach Anspruch 11, dadurch gekennzeichnet, daß in den Schritt b) als monoklonaler Antikörper der Antikörper eingesetzt wird, der das in Figur 3 dargestellte Reaktionsmuster gegenüber intaktem Prokollagen (Typ III) und pN-Kollagen (Peak 4a), intaktem aminoterminalen Prokollagen Peptid (Typ III) (Peak 5a) sowie Col 1 und Abbauprodukten des aminoterminalen Prokollagen Peptids (Typ III) mit gleichem Molekulargewicht wie Col 1 (Peak 6a) aufweist.

14. Verfahren nach Anspruch 12, dadurch gekennzeichnet, daß in den Schritt a) als monoklonaler Antikörper der Antikörper eingesetzt wird, der das in Figur 3 dargestellte Reaktionsmuster gegenüber intaktem Prokollagen (Typ III) und pN-Kollagen (Peak 4a), intaktem aminoterminalen Prokollagen Peptid (Typ III) (Peak 5a) sowie Col 1 und Abbauprodukten des aminoterminalen Prokollagen Peptids (Typ III) mit gleichem Molekulargewicht wie Col 1 (Peak 6a) aufweist.

15. Verfahren nach Anspruch 13 oder 14, dadurch gekennzeichnet, daß als monoklonaler Antikörper PIIIP 296 der Hybridoma Zellinie ECACC 87042308 eingesetzt wird.

16. Eine diagnostische Zusammensetzung zur Feststellung der Prokollagen Peptid (Typ III)-Menge in Körperflüssigkeiten, gekennzeichnet durch eine wirksame Menge des monoklonalen Antikörpers nach einem oder mehreren der Ansprüche 1 bis 5, alleine oder in Kombination mit anderen Antikörpern, im Gemisch mit einem diagnostisch annehmbaren Träger.

## Patentansprüche (Patentansprüche für folgende(n) Vertragsstaat(en): ES, GR)

1. Monoklonaler Antikörper mit dem in Fig. 2 dargestellten Reaktionsmuster gegenüber intaktem Prokollagen (Typ III) und pN-Kollagen (Peak 1a), intaktem aminoterminalem Prokollagen Peptid (Typ III) (Peak 2a), Abbauprodukten des aminoterminalen Prokollagen Peptids (Typ III), die mit ihrem Molekulargewicht zwischen dem des aminoterminalen Prokollagen Peptids (Typ III) und dem des Col 1 liegen (Peak 3a), sowie Col 1 und Abbauprodukten des aminoterminalen Prokollagen Peptids Typ III mit gleichem Molekulargewicht wie Col 1 (Peak 4a).

2. Monoklonaler Antikörper nach Anspruch 1 mit spezifischer Wirkung gegen ein Epitop des aminoterminalen Prokollagen Peptids (Typ III), das in Col 1 nicht vorhanden ist.

3. Monoklonaler Antikörper nach Anspruch 1 oder 2, dadurch gekennzeichnet, daß er der Klasse IgG angehört.

4. Monoklonaler Antikörper nach einem oder mehreren der Ansprüche 1 bis 3, dadurch gekennzeichnet, daß er durch ein Hybridom gebildet wird, das durch Fusion von Zellen einer Myelom Linie mit Lymphozyten eines zuvor mit aminoterminalem Prokollagen Peptid (Typ III) immunisierten Tieres entstanden ist.

5. Monoklonaler Antikörper PIIIP 226 der Hybridoma Zellinie ECACC 88030202.

6. Hybridoma Zellinie, die einen Antikörper gemäß der Ansprüche 1 bis 5 produziert, gebildet durch Fusion von Zellen aus einer Myelom-Zellinie und Lymphozyten eines zuvor mit Prokollagen Peptid (Typ III) immunisierten Tieres.

7. Die Hybridoma Zellinie ECACC 88030202.

8. Ein Verfahren zur Herstellung des monoklonalen Antikörpers dem in Figur 2 dargestellten Reaktionsmuster gegenüber intaktem Prokollagen (Typ III) und pN-Kollagen (Peak 1a), intaktem aminoterminalen Prokollagen Peptid (Typ III) (Peak 2a) Abbauprodukten des aminoterminalen Prokollagen Peptids (Typ III), die mit ihrem Molekulargewicht zwischen dem des aminoterminalen Prokollagen Peptids (Typ III) und dem des Col 1 liegen (Peak 3a), sowie Col 1 und Abbauprodukten des aminoterminalen Prokollagen Peptids Typ III mit gleichem Molekulargewicht wie Col 1 (Peak 4a), dadurch gekennzeichnet, daß
a) Tiere mit aminoterminalem Prokollagen Peptid (Typ III) immunisiert werden,
b) Lymphozyten gewonnen und mit Myelom-Zellen fusioniert werden,
c) die Hybride hinsichtlich des Vorliegens eines Antikörpers mit den in einem der Ansprüche 1 bis 3 angegebenen Eigenschaften ausgewählt und kloniert werden und
d) der Antikörper aus den genannten Klonen gewonnen wird.

9. Verfahren nach Anspruch 8, dadurch gekennzeichnet, daß zur Ausführung des Schritts d) die Hybridoma Zellinie ECACC 88030202 eingesetzt wird.

10. Verfahren zur quantitativen immunologischen Bestimmung von Prokollagen Peptid (Typ III) mit Antikörpern, dadurch gekennzeichnet, daß
a) eine flüssige Probe, die aminoterminales Prokollagen Peptid (Typ III) oder Prokollagen (Typ III) enthält, mit dem monoklonalen Antikörper mit dem in Fig. 2 dargestellten Reaktionsmuster gegenüber intaktem Prokollagen (Typ III) und pN-Kollagen (Peak 1a), intaktem aminoterminalem Prokollagen Peptid (Typ III) (Peak 2a), Abbauprodukten des aminoterminalen Prokollagen Peptids (Typ III), die mit ihrem Molekulargewicht zwischen dem des aminotemrinalen Prokollagen Peptids (Typ III) und dem des Col 1 liegen (Peak 3a), sowie Col 1 und Abbauprodukten des aminoterminalen Prokollagen Peptids Typ III mit gleichem Molekulargewicht wie Col 1 (Peak 4a) zur Reaktion gebracht wird und
b) die Menge des aminoterminalen Prokollagen Peptids (Typ III) bzw. des Prokollagens (Typ III) über den gebildeten Antigen-Antikörper-Komplex bestimmt wird.

11. Verfahren zur quantitativen immunologischen Bestimmung von Prokollagen Peptid (Typ III) mit Antikörpern, dadurch gekennzeichnet, daß
a) der monoklonale Antikörper mit dem in Fig. 2 dargestellten Reaktionsmuster gegenüber intaktem Prokollagen (Typ III) und pN-Kollagen (Peak 1a), intaktem aminoterminalem Prokollagen Peptid (Typ III) (Peak 2a), Abbauprodukten des aminoterminalen Prokollagen Peptids (Typ III), die mit ihrem Molekulargewicht zwischen dem des aminoterminalen Prokollagen Peptids (Typ III) und dem des Col 1 liegen (Peak 3a), sowie Col 1 und Abbauprodukten des aminoterminalen Prokollagen Peptids Typ III mit gleichem Molekulargewicht wie Col 1 (Peak 4a) an eine feste Matrix gekuppelt wird,
b) eine flüssige Probe, die aminoterminales Prokollagen Peptid (Typ III) oder Prokollagen (Typ III) enthält, mit dem genannten Antikörper zur Reaktion gebracht wird und
c) das gebundene Antigen durch markierte monoklonale oder polyklonale Antikörper mit Spezifität für Prokollagen Peptid (Typ III) oder Prokollagen (Typ III) nachgewiesen wird.

12. Verfahren nach Anspruch 11, dadurch gekennzeichnet, daß in den Schritt a) polyklonale oder monoklonale Antikörper mit Spezifität für Prokollagen Peptid (Typ III) und/oder Prokollagen (Typ III) und in den Schritt c) der markierte monoklonale Antikörper mit dem in Fig. 2 dargestellten Reaktionsmuster gegenüber intaktem Prokollagen (Typ III) und pN-Kollagen (Peak 1a), intaktem aminoterminalem Prokollagen Peptid (Typ III) (Peak 2a), Abbauprodukten des aminoterminalen Prokollagen Peptids (Typ III), die mit ihrem Molekulargewicht zwischen dem des aminoterminalen Prokollagen Peptids (Typ III) und dem des Col 1 liegen (Peak 3a), sowie Col 1 und Abbauprodukten des aminoterminalen Prokollagen Peptids Typ III mit gleichem Molekulargewicht wie Col 1 (Peak 4a) eingesetzt werden.

13. Verfahren nach Anspruch 11, dadurch gekennzeichnet, daß in den Schritt b) als monoklonaler Antikörper der Antikörper eingesetzt wird, der das in Figur 3 dargestellte Reaktionsmuster gegenüber intaktem Prokollagen (Typ III) und pN-Kollagen (Peak 4a), intaktem aminoterminalen Prokollagen Peptid (Typ III) (Peak 5a) sowie Col 1 und Abbauprodukten des aminoterminalen Prokollagen Peptids (Typ III) mit gleichem Molekulargewicht wie Col 1 (Peak 6a) aufweist.

14. Verfahren nach Anspruch 12, dadurch gekennzeichnet, daß in den Schritt a) als monoklonaler Antikörper der Antikörper eingesetzt wird, der das in Figur 3 dargestellte Reaktionsmuster gegenüber intaktem Prokollagen (Typ III) und pN-Kollagen (Peak 4a), intaktem aminoterminalen Prokollagen Peptid (Typ III) (Peak 5a) sowie Col 1 und Abbauprodukten des aminoterminalen Prokollagen Peptids (Typ III) mit gleichem Molekulargewicht wie Col 1 (Peak 6a) aufweist.

15. Verfahren nach Anspruch 13 oder 14, dadurch gekennzeichnet, daß als monoklonaler Antikörper PIIIP 296 der Hybridoma Zellinie ECACC 87042308 eingesetzt wird.

16. Eine diagnostische Zusammensetzung zur Feststellung der Prokollagen Peptid (Typ III)-Menge in Körperflüssigkeiten, gekennzeichnet durch eine wirksame Menge des monoklonalen Antikörpers nach einem oder mehreren der Ansprüche 1 bis 5, alleine oder in Kombination mit anderen Antikörpern, im Gemisch mit einem diagnostisch annehmbaren Träger.

## Claims (Claims for the following Contracting State(s): AT, BE, CH, DE, FR, GB, IT, LI, LU, NL, SE)

1. A monoclonal antibody having the reaction pattern depicted in Fig. 2 towards intact procollagen (type III) and pN-collagen (peak 1a), intact amino-terminal procollagen peptide (type III) (peak 2a), degradation products of amino-terminal procollagen peptide (type III) whose molecular weights are between that of amino-terminal procollagen peptide (type III) and that of col 1 (peak 3a), as well as col 1 and degradation products of aminoterminal procollagen peptide (type III) with the same molecular weight as col 1 (peak 4a).

2. A monoclonal antibody as claimed in claim 1, having a specific action against an epitope of amino-terminal procollagen peptide (type III) which is not present in col 1.

3. A monoclonal antibody as claimed in claim 1 or 2, which belongs to the IgG class.

4. A monoclonal antibody as claimed in one or more of claims 1 to 3, which is formed by a hybridoma which is produced by fusion of cells of a myeloma line with lymphocytes from an animal which has previously been immunized with amino-terminal procollagen peptide (type III).

5. The monoclonal antibody PIIIP 226 of the hybridoma cell line ECACC 88030202.

6. A hybridoma cell line which produces an antibody as claimed in any of claims 1 to 5, formed by fusion of cells from a myeloma cell line and lymphocytes from an animal which has previously been immunized with procollagen peptide (type III).

7. The hybridoma cell line ECACC 88030202.

8. A process for the preparation of a monoclonal antibody as claimed in one or more of claims 1 to 5, which comprises
a) animals being immunized with amino-terminal procollagen peptide (type III)
b) lymphocytes being obtained and fused with myeloma cells
c) the hybrids being selected for the presence of an antibody having the properties indicated in one of claims 1 to 3, and being cloned, and
d) the antibody being obtained from the said clones.

9. The process as claimed in claim 8, wherein the hybridoma cell line ECACC 88030202 is employed to carry out step d).

10. A method for the quantitative immunological determination of procollagen peptide (type III) using antibodies, which comprises
a) a liquid sample which contains amino-terminal procollagen peptide (type III) or procollagen (type III) being reacted with the monoclonal antibody as claimed in one or more of claims 1 to 5, and
b) the amount of amino-terminal procollagen peptide (type III) or of procollagen (type III) being determined via the antigen-antibody complex formed.

11. A method for the quantitative immunological determination of procollagen peptide (type III) using antibodies, which comprises
a) the monoclonal antibody as claimed in one or more of claims 1 to 5 being coupled to a solid matrix
b) a liquid sample which contains amino-terminal procollagen peptide (type III) or procollagen (type III) being reacted with the said antibody, and
c) the bound antigen being detected by labeled monoclonal or polyclonal antibodies with specificity for procollagen peptide (type III) or procollagen (type III).

12. The method as claimed in claim 11, wherein polyclonal or monoclonal antibodies with specificity for procollagen peptide (type III) and/or procollagen (type III) are employed in step a), and the labeled monoclonal antibody as claimed in one or more of claims 1 to 5 is employed in step c).

13. The method as claimed in claim 11, wherein the monoclonal antibody employed in step b) is the antibody which has the reaction pattern depicted in Figure 3 towards intact procollagen (type III) end pN-collagen (peak 4a), intact amino-terminal procollagen peptide (type III) (peak 5a) as well as col 1 and degradation products of amino-terminal procollagen peptide (type III) with the same molecular weight as col 1 (peak 6a).

14. The method as claimed in claim 12, wherein the monoclonal antibody employed in step a) is the antibody which has the reaction pattern depicted in Figure 3 towards intact procollagen (type III) and pN-collagen (peak 4a), intact amino-terminal procollagen peptide (type III) (peak 5a) as well as col 1 and degradation products of amino-terminal procollagen peptide (type III) with the same molecular weight as col 1 (peak 6a).

15. The method as claimed in claim 13 or 14, wherein the monoclonal antibody employed is PIIIP 296 of the hybridoma cell line ECACC 87042308.

16. A diagnostic composition for establishing the amount of procollagen peptide (type III) in body fluids, which contains an effective amount of the monoclonal antibody as claimed in one or more of claims 1 to 5, alone or in combination with other antibodies, mixed with a carrier acceptable in diagnosis.

## Claims (Claims for the following Contracting State(s): ES, GR)

1. A monoclonal antibody having the reaction pattern depicted in Fig. 2 towards intact procollagen (type III) and pN-collagen (peak 1a), intact amino-terminal procollagen peptide (type III) (peak 2a), degradation products of amino-terminal procollagen peptide (type III) whose molecular weights are between that of amino-terminal procollagen peptide (type III) and that of col 1 (peak 3a), as well as col 1 and degradation products of aminoterminal procollagen peptide (type III) with the same molecular weight as col 1 (peak 4a).

2. A monoclonal antibody as claimed in claim 1, having a specific action against an epitope of amino-terminal procollagen peptide (type III) which is not present in col 1.

3. A monoclonal antibody as claimed in claim 1 or 2, which belongs to the IgG class.

4. A monoclonal antibody as claimed in one or more of claims 1 to 3, which is formed by a hybridoma which is produced by fusion of cells of a myeloma line with lymphocytes from an animal which has previously been immunized with amino-terminal procollagen peptide (type III).

5. The monoclonal antibody PIIIP 226 of the hybridoma cell line ECACC 88030202.

6. A hybridoma cell line which produces an antibody as claimed in any of claims 1 to 5, formed by fusion of cells from a myeloma cell line and lymphocytes from an animal which has previously been immunized with procollagen peptide (type III).

7. The hybridoma cell line ECACC 88030202.

8. A process for the preparation of the monoclonal antibody having the reaction pattern depicted in Fig. 2 towards intact procollagen (type III) and pN-collagen (peak 1a), intact amino-terminal procollagen peptide (type III) (peak 2a), degradation products of amino-terminal procollagen peptide (type III) whose molecular weights are between that of amino-terminal procollagen peptide (type III) and that of col 1 (peak 3a), as well as col 1 and degradation products of aminoterminal procollagen peptide (type III) with the same molecular weight as col 1 (peak 4a), which comprises
a) animals being immunized with amino-terminal procollagen peptide (type III)
b) lymphocytes being obtained and fused with myeloma cells
c) the hybrids being selected for the presence of an antibody having the properties indicated in one of claims 1 to 3, and being cloned, and
d) the antibody being obtained from the said clones.

9. The process as claimed in claim 8, wherein the hybridoma cell line ECACC 88030202 is employed to carry out step d).

10. A method for the quantitative immunological determination of procollagen peptide (type III) using antibodies, which comprises
a) a liquid sample which contains amino-terminal procollagen peptide (type III) or procollagen (type III) being reacted with the monoclonal antibody having the reaction pattern depicted in Fig. 2 towards intact procollagen (type III) and pN-collagen (peak 1a), intact amino-terminal procollagen peptide (type III) (peak 2a), degradation products of amino-terminal procollagen peptide (type III) whose molecular weights are between that of amino-terminal procollagen peptide (type III) and that of col 1 (peak 3a), as well as col 1 and degradation products of aminoterminal procollagen peptide (type III) with the same molecular weight as col 1 (peak 4a), and
b) the amount of amino-terminal procollagen peptide (type III) or of procollagen (type III) being determined via the antigen-antibody complex formed.

11. A method for the quantitative immunological determination of procollagen peptide (type III) using antibodies, which comprises
a) the monoclonal antibody having the reaction pattern depicted in Fig. 2 towards intact procollagen (type III) and pN-collagen (peak 1a), intact amino- terminal procollagen peptide (type III) (peak 2a), degradation products of amino-terminal procollagen peptide (type III) whose molecular weights are between that of amino-terminal procollagen peptide (type III) and that of col 1 (peak 3a), as well as col 1 and degradation products of aminoterminal procollagen peptide (type III) with the same molecular weight as col 1 (peak 4a) being coupled to a solid matrix
b) a liquid sample which contains amino-terminal procollagen peptide (type III) or procollagen (type III) being reacted with the said antibody, and
c) the bound antigen being detected by labeled monoclonal or polyclonal antibodies with specificity for procollagen peptide (type III) or procollagen (type III).

12. The method as claimed in claim 11, wherein polyclonal or monoclonal antibodies with specificity for procollagen peptide (type III) and/or procollagen (type III) are employed in step a), and the labeled monoclonal antibody having the reaction pattern depicted in Fig. 2 towards intact procollagen (type III) and pN-collagen (peak 1a), intact amino-terminal procollagen peptide (type III) (peak 2a), degradation products of amino-terminal procollagen peptide (type III) whose molecular weights are between that of amino-terminal procollagen peptide (type III) and that of col 1 (peak 3a), as well as col 1 and degradation products of aminoterminal procollagen peptide (type III) with the same molecular weight as col 1 (peak 4a) is employed in step c).

13. The method as claimed in claim 11, wherein the monoclonal antibody employed in step b) is the antibody which has the reaction pattern depicted in Figure 3 towards intact procollagen (type III) and pN-collagen (peak 4a), intact amino-terminal procollagen peptide (type III) (peak 5a) as well as col 1 and degradation products of amino-terminal procollagen peptide (type III) with the same molecular weight as col 1 (peak 6a).

14. The method as claimed in claim 12, wherein the monoclonal antibody employed in step a) is the antibody which has the reaction pattern depicted in Figure 3 towards intact procollagen (type III) and pN-collagen (peak 4a), intact amino-terminal procollagen peptide (type III) (peak 5a) as well as col 1 and degradation products of amino-terminal procollagen peptide (type III) with the same molecular weight as col 1 (peak 6a).

15. The method as claimed in claim 13 or 14, wherein the monoclonal antibody employed is PIIIP 296 of the hybridoma cell line ECACC 87042308.

16. A diagnostic composition for establishing the amount of procollagen peptide (type III) in body fluids, which contains an effective amount of the monoclonal antibody as claimed in one or more of claims 1 to 5, alone or in combination with other antibodies, mixed with a carrier acceptable in diagnosis.

## Revendications (Revendications pour l'(les) Etat(s) contractant(s) suivant(s): AT, BE, CH, DE, FR, GB, IT, LI, LU, NL, SE)

1. Anticorps monoclonal ayant le profil réactionnel représenté sur la figure 2, vis-à-vis du procollagène (type III) intact et du pN-collagène (pic 1a), du peptide amino-terminal intact du procollagène (type III) (pic 2a), des produits de dégradation du peptide amino-terminal du procollagène (type III), dont la masse moléculaire se situe entre celle du peptide amino-terminal du procollagène (type III) et celle du Col 1 (pic 3a), ainsi que du Col 1 et des produits de dégradation du peptide amino-terminal du procollagène de type III, ayant la même masse moléculaire que le Col 1 (pic 4a).

2. Anticorps monoclonal selon la revendication 1, à activité spécifique contre un épitope du peptide amino-terminal du procollagène (type III) qui n'est pas présent dans le Col 1.

3. Anticorps monoclonal selon la revendication 1 ou 2, caractérisé en ce qu'il appartient à la classe des IgG.

4. Anticorps monoclonal selon une ou plusieurs des revendications 1 à 3, caractérisé en ce qu'il est produit par un hybridome qui est formé par fusion de cellules d'une lignée de myélome avec des lymphocytes d'un animal préalablement immunisé à l'aide du peptide amino-terminal du procollagène (type III).

5. Anticorps monoclonal PIIIP 226 de la lignée cellulaire d'hybridomes ECACC 88030202.

6. Lignée cellulaire d'hybridomes produisant un anticorps selon les revendications 1 à 5, qui est formée par fusion de cellules provenant d'une lignée cellulaire de myélome et de lymphocytes d'un animal préalablement immunisé à l'aide du peptide du procollagène (type III).

7. Lignée cellulaire d'hybridomes ECACC 88030202.

8. Procédé pour la production d'un anticorps monoclonal selon une ou plusieurs des revendications 1 à 5, caractérisé en ce que
a) on immunise des animaux à l'aide du peptide amino-terminal du procollagène (type III),
b) on recueille des lymphocytes et on les fait fusionner avec des cellules de myélome,
c) on sélectionne les hybrides sur la base de la présence d'un anticorps ayant les propriétés indiquées dans les revendications 1 à 3, et on les soumet à un clonage, et
d) on recueille l'anticorps à partir desdits clones.

9. Procédé selon la revendication 8, caractérisé en ce que, pour l'exécution de l'étape d), on utilise la lignée cellulaire d'hybridomes ECACC 88030202.

10. Procédé pour la détermination immunologique quantitative du peptide du procollagène (type III) à l'aide d'anticorps, caractérisé en ce que
a) on met en réaction un échantillon liquide, qui contient du procollagène (type III) ou le peptide amino-terminal du procollagène (type III), avec l'anticorps monoclonal selon une ou plusieurs des revendications 1 à 5, et
b) on détermine la quantité du procollagène (type III) ou du peptide amino-terminal du procollagène (type III) au moyen du complexe antigène-anticorps formé.

11. Procédé pour la détermination immunologique quantitative du peptide du procollagène (type III) à l'aide d'anticorps, caractérisé en ce que
a) on lie à une matrice solide l'anticorps monoclonal selon une ou plusieurs des revendications 1 à 5,
b) on met en réaction avec ledit anticorps un échantillon liquide qui contient du procollagène (type III) ou le peptide amino-terminal du procollagène (type III), et
c) l'antigène fixé est détecté au moyen d'anticorps monoclonaux ou polyclonaux marqués, à spécificité pour le procollagène (type III) ou pour le peptide du procollagène (type III).

12. Procédé selon la revendication 11, caractérisé en ce que, dans l'étape a), on utilise des anticorps monoclonaux ou polyclonaux à spécificité pour le procollagène (type III) et/ou pour le peptide du procollagène (type III), et, dans l'étape c), on utilise l'anticorps monoclonal marqué, selon une ou plusieurs des revendications 1 à 5.

13. Procédé selon la revendication 11, caractérisé en ce que, dans l'étape b), on utilise comme anticorps monoclonal l'anticorps qui présente le profil réactionnel représenté sur la figure 3, vis-à-vis du procollagène (type III) intact et du pN-collagène (pic 4a), du peptide amino-terminal intact du procollagène (type III) (pic 5a), ainsi que du Col 1 et des produits de dégradation du peptide amino-terminal du procollagène (type III), ayant la même masse moléculaire que le Col 1 (pic 6a).

14. Procédé selon la revendication 12, caractérisé en ce que, dans l'étape a), on utilise comme anticorps monoclonal l'anticorps qui présente le profil réactionnel représenté sur la figure 3, vis-à-vis du procollagène (type III) intact et du pN-collagène (pic 4a), du peptide amino-terminal intact du procollagène (type III) (pic 5a), ainsi que du Col 1 et des produits de dégradation du peptide amino-terminal du procollagène (type III), ayant la même masse moléculaire que le Col 1 (pic 6a).

15. Procédé selon la revendication 13 ou 14, caractérisé en ce que, comme anticorps monoclonal, on utilise l'anticorps PIIIP 296 de la lignée cellulaire d'hybridomes ECACC 87042308.

16. Composition diagnostique pour la détermination de la quantité de peptide du procollagène (type III) dans des liquides corporels, caractérisée par une quantité efficace de l'anticorps monoclonal selon une ou plusieurs des revendications 1 à 5, seule ou en association avec d'autres anticorps, en mélange avec un véhicule acceptable du point de vue diagnostique.

## Revendications (Revendications pour l'(les) Etat(s) contractant(s) suivant(s): ES, GR)

1. Anticorps monoclonal ayant le profil réactionnel représenté sur la figure 2, vis-à-vis du procollagène (type III) intact et du pN-collagène (pic 1a), du peptide amino-terminal intact du procollagène (type III) (pic 2a), des produits de dégradation du peptide amino-terminal du procollagène (type III), dont la masse moléculaire se situe entre celle du peptide amino-terminal du procollagène (type III) et celle du Col 1 (pic 3a), ainsi que du Col 1 et des produits de dégradation du peptide amino-terminal du procollagène de type III, ayant la même masse moléculaire que le Col 1 (pic 4a).

2. Anticorps monoclonal selon la revendication 1, à activité spécifique contre un épitope du peptide amino-terminal du procollagène (type III) qui n'est pas présent dans le Col 1.

3. Anticorps monoclonal selon la revendication 1 ou 2, caractérisé en ce qu'il appartient à la classe des IgG.

4. Anticorps monoclonal selon une ou plusieurs des revendications 1 à 3, caractérisé en ce qu'il est produit par un hybridome qui est formé par fusion de cellules d'une lignée de myélome avec des lymphocytes d'un animal préalablement immunisé à l'aide du peptide amino-terminal du procollagène (type III).

5. Anticorps monoclonal PIIIP 226 de la lignée cellulaire d'hybridomes ECACC 88030202.

6. Lignée cellulaire d'hybridomes produisant un anticorps selon les revendications 1 à 5, qui est formée par fusion de cellules provenant d'une lignée cellulaire de myélome et de lymphocytes d'un animal préalablement immunisé à l'aide du peptide du procollagène (type III).

7. Lignée cellulaire d'hybridomes ECACC 88030202.

8. Procédé pour la production de l'anticorps monoclonal ayant le profil réactionnel représenté sur la figure 2, vis-à-vis du procollagène (type III) intact et du pN-collagène (pic 1a), du peptide aminoterminal intact du procollagène (type III) (pic 2a), des produits de dégradation du peptide amino-terminal du procollagène (type III), dont la masse moléculaire se situe entre celle du peptide amino-terminal du procollagène (type III) et celle du Col 1 (pic 3a), ainsi que du Col 1 et des produits de dégradation du peptide amino-terminal du procollagène de type III, ayant la même masse moléculaire que le Col 1, (pic 4a), caractérisé en ce que
a) on immunise des animaux à l'aide du peptide amino-terminal du procollagène (type III),
b) on recueille des lymphocytes et on les fait fusionner avec des cellules de myélome,
c) on sélectionne les hybrides sur la base de la présence d'un anticorps ayant les propriétés indiquées dans les revendications 1 à 3, et on les soumet à un clonage, et
d) on recueille l'anticorps à partir desdits clones.

9. Procédé selon la revendication 8, caractérisé en ce que, pour l'exécution de l'étape d), on utilise la lignée cellulaire d'hybridomes ECACC 88030202.

10. Procédé pour la détermination immunologique quantitative du peptide du procollagène (type III) à l'aide d'anticorps, caractérisé en ce que
a) on met en réaction un échantillon liquide, qui contient du procollagène (type III) ou le peptide amino-terminal du procollagène (type III), avec l'anticorps monoclonal ayant le profil réactionnel représenté sur la figure 2, vis-à-vis du procollagène (type III) intact et du pN-collagène (pic 1a), du peptide aminoterminal intact du procollagène (type III) (pic 2a), des produits de dégradation du peptide amino-terminal du procollagène (type III), dont la masse moléculaire se situe entre celle du peptide amino-terminal du procollagène (type III) et celle du Col 1 (pic 3a), ainsi que du Col 1 et des produits de dégradation du peptide amino-terminal du procollagène de type III, ayant la même masse moléculaire que le Col 1 (pic 4a), et
b) on détermine la quantité du procollagène (type III) ou du peptide amino-terminal du procollagène (type III) au moyen du complexe antigène-anticorps formé.

11. Procédé pour la détermination immunologique quantitative du peptide du procollagène (type III) à l'aide d'anticorps, caractérisé en ce que
a) on lie à une matrice solide l'anticorps monoclonal ayant le profil réactionnel représenté sur la figure 2, vis-à-vis du procollagène (type III) intact et du pN-collagène (pic 1a), du peptide aminoterminal intact du procollagène (type III) (pic 2a), des produits de dégradation du peptide amino-terminal du procollagène (type III), dont la masse moléculaire se situe entre celle du peptide amino-terminal du procollagène (type III) et celle du Col 1 (pic 3a), ainsi que du Col 1 et des produits de dégradation du peptide amino-terminal du procollagène de type III, ayant la même masse moléculaire que le Col 1 (pic 4a),
b) on met en réaction avec ledit anticorps un échantillon liquide qui contient du procollagène (type III) ou le peptide amino-terminal du procollagène (type III), et
c) l'antigène fixé est détecté au moyen d'anticorps monoclonaux ou polyclonaux marqués, à spécificité pour le procollagène (type III) ou pour le peptide du procollagène (type III).

12. Procédé selon la revendication 11, caractérisé en ce que, dans l'étape a), on utilise des anticorps monoclonaux ou polyclonaux à spécificité pour le procollagène (type III) et/ou pour le peptide du procollagène (type III), et, dans l'étape c), on utilise l'anticorps monoclonal marqué, ayant le profil réactionnel représenté sur la figure 2, vis-à-vis du procollagène (type III) intact et du pN-collagène (pic 1a), du peptide aminoterminal intact du procollagène (type III) (pic 2a), des produits de dégradation du peptide amino-terminal du procollagène (type III), dont la masse moléculaire se situe entre celle du peptide amino-terminal du procollagène (type III) et celle du Col 1 (pic 3a), ainsi que du Col 1 et des produits de dégradation du peptide amino-terminal du procollagène de type III, ayant la même masse moléculaire que le Col 1 (pic 4a).

13. Procédé selon la revendication 11, caractérisé en ce que, dans l'étape b), on utilise comme anticorps monoclonal l'anticorps qui présente le profil réactionnel représenté sur la figure 3, vis-à-vis du procollagène (type III) intact et du pN-collagène (pic 4a), du peptide amino-terminal intact du procollagène (type III) (pic 5a), ainsi que du Col 1 et des produits de dégradation du peptide amino-terminal du procollagène (type III), ayant la même masse moléculaire que le Col 1 (pic 6a).

14. Procédé selon la revendication 12, caractérisé en ce que, dans l'étape a), on utilise comme anticorps monoclonal l'anticorps qui présente le profil réactionnel représenté sur la figure 3, vis-à-vis du procollagène (type III) intact et du pN-collagène (pic 4a), du peptide amino-terminal intact du procollagène (type III) (pic 5a), ainsi que du Col 1 et des produits de dégradation du peptide amino-terminal du procollagène (type III), ayant la même masse moléculaire que le Col 1 (pic 6a).

15. Procédé selon la revendication 13 ou 14, caractérisé en ce que, comme anticorps monoclonal, on utilise l'anticorps PIIIP 296 de la lignée cellulaire d'hybridomes ECACC 87042308.

16. Composition diagnostique pour la détermination de la quantité de peptide du procollagène (type III) dans des liquides corporels, caractérisée par une quantité efficace de l'anticorps monoclonal selon une ou plusieurs des revendications 1 à 5, seule ou en association avec d'autres anticorps, en mélange avec un véhicule acceptable du point de vue diagnostique.
